# EUROPEAN PATENT APPLICATION

(11) **EP 2 548 610 A1**
(43) Date of publication of application: **23.01.2013**
(21) Application number: 12177372.5
(22) Date of filing: 20.07.2012
(51) Int. Cl.: A61N 1/32

(54) **Apparatus for the aesthetic treatment of a patient**

(30) Priority: 20.07.2011 IT MI20111356
(71) Applicant: Mac Pharma S.r.l., 31100 Treviso (TR) (IT)
(72) Inventor: Massimo, Chimenti, 31021 Mogliano Veneto TV (IT)
(74) Representative: Bonvicini, Davide

(57) **Abstract**

The present invention concerns an apparatus (1) for cosmetic skin treatment of a user, which comprises an applicator (100) having at least one base body (105) mainly extending in a longitudinal direction (X-X) and equipped with a first (111) and a second (110) unpolarized electrodes, each adapted to contact an area of the patient's skin to be treated, to apply a pulsed electric field (E) into a predetermined skin volume, a power source (10) connected to the electrodes (110, 111) for generating pulsed voltage (Vₐ) to generate the electric field (E) and a transformer (T) connected between the electrodes (110, 111) and the power source (10). The base body (10) also comprises a reservoir (120) for containing skin treatment substances, pumping means (122) associated with the reservoir (120) for discharging the substances from said applicator (100). Preferably, the reservoir (120) and the pumping means (122) are entirely held in the base body (105).

## Description

The present invention relates to an apparatus for cosmetic skin treatment of a patient as defined in the preamble of claim 1. Particularly, the present invention provides an apparatus that can introduce particular curative substances into the skin by electroporation.

Electroporation techniques are known in the art and include introduction of molecules into cells through the cell membrane. For instance, inorganic molecules (drugs, chemicals or therapeutic substances) or organic molecules (DNA) may be introduced, depending on the treatment to be conducted.

Many types of manipulators are available, but they are inconvenient to use by the medical staff.

An example of these is the manipulator of US 2007/0135755, which discloses a system, a method and an apparatus for enhancing subcutaneous absorption of curative substances for cosmetic skin treatment. Namely, this apparatus is designed to apply a sequence of pulses by a generator using an array of electrodes that is placed in contact with the skin through a vibrating plate. The electric pulses applied by the electrodes are obtained by discharging a capacitor or by using a transformer adapted to control the maximum value of current applied to the skin to be treated. A liquid or gel container is placed between the vibrating plate and the electrodes, for pouring the substance on the skin. A roller supported by the container allows application of gel, while the applicator is moved on a patient's skin in controlled fashion. In one embodiment, gel application is provided by a manually handled external syringe.

FR 2 440 744 discloses a device for electrotherapeutic applications, having an applicator with a removable and interchangeable terminal. The applicator comprises two tubes connected to an external reservoir, for conveying the curative gels using an external pump. The applicator may be of mono- or bipolar applicator, which is powered by a DC source controlled by a pulse generator.

US 5 911 223 discloses an electroporation applicator with an incorporated gel reservoir, with a pulger/piston associated therewith, whose free end is external to the applicator, allowing manipulation by an operator. A voltage generator is designed to send a pulsed voltage between the first electrode and the second electrode to generate a localized electric field in the epidermis. The first electrode has a plurality of through holes for the passage of the curative substance contained in the reservoir. In one embodiment, an additional electrode is provided, for increasing current flow through the epidermis. Thus, current flows from the applicator to the electrode and expands the area covered by the electric field.

Nevertheless, the above solutions have the drawback of requiring manual control of curative substance delivery or reservoir filling, without the possibility of optimizing the electroporation process.

The need arises from the above for a cosmetic skin treatment apparatus that allows quick and controlled administration of substances into the cells.

This purpose is fulfilled by an apparatus for cosmetic skin treatment of a patient, as defined in claim 1.

Further characteristics and advantages of the apparatus for cosmetic skin treatment of a patient according to the present invention will result from the following description of certain preferred exemplary embodiments thereof, which are given by way of illustration and without limitation, with reference to the accompanying figures, in which:
- Figure 1 shows a sectional view of a first embodiment of the apparatus of the invention, and a diagram of the power circuit;
- Figure 2 shows a sectional view of a second embodiment of the apparatus of the invention.

Referring to the accompanying figures, numeral 1 generally designates an apparatus for cosmetic skin treatment of a patient according to the present invention.

The apparatus 1 comprises an applicator 100 which is designed to be held in the hand of a user specialized for cosmetic skin treatment of patients. Particularly, the applicator 100 has a base body 105 mainly extending in a longitudinal direction X-X and having a preferably cylindrical shape, pointed at one terminal end thereof 105a, like a pen.

The apparatus 1 comprises a power source 10 for supplying power to a pair of electrodes 110, 111 located in the applicator 100 at the terminal end 105a of the base body.

Preferably, the base body 105 tapers toward its terminal end 105a, and hence toward the electrodes.

Particularly, the applicator 100 has a first electrode 110 and a second electrode 111, each adapted to contact a predetermined area of the patient's epidermis.

The power source 10 is connected to the pair of electrodes 110, 111 and generates a pulsed voltage Vₐ.

The potential difference at the ends of the electrodes 110, 111 allows an electric field E to be applied as soon as the applicator 100 contacts the skin of the patient. In practice, the electric field E generated between the two electrodes 110, 111 penetrates the skin to make the cell membrane permeable.

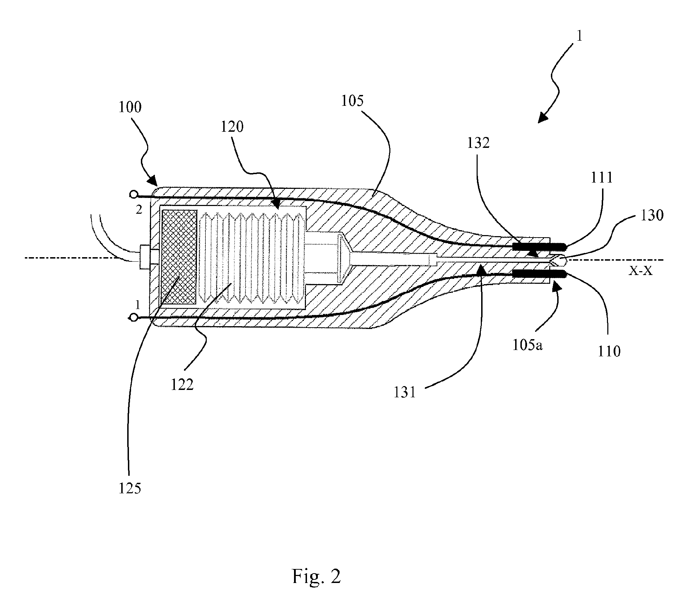

Referring to the example as shown in Figure 2, the apparatus 1 is equipped with a transformer T connected between the pair of electrodes 110, 111 and the power source 10.

Advantageously, the secondary winding T_{b} of the transformer T is in direct signal communication with the pair of electrodes 110, 111. Particularly, the transformer T has a first terminal of the secondary winding (T_{b}) in direct signal communication with the first electrode 110 of the applicator 100 and a second terminal of the secondary winding T_{b} in direct signal communication with the second electrode 111 of the applicator. Thus, both electrodes (namely the part directly applied to the patient) can become unpolarized and hence insulated from any other part of the apparatus, without requiring any grounding to limit, in case of failure (e.g. when an undesired voltage from an external source is applied to the patient between the applied part and the ground), the admissible leakage current on the patient, as required by the standard IEC/EN 60601-1.

Therefore, the provision of a pair of unpolarized electrodes 110, 111 allows a variable electric field E to be applied in the skin of a patient, thereby preventing any alteration of the physico-chemical properties of the curative substance to be applied to the patient.

In one embodiment, each electrode at least partially projects out of the terminal end 105a of the base body 105 in a direction substantially parallel to the longitudinal direction of extension X-X.

In one version, the first electrode 110 is spaced from the second electrode 111 at a distance d₁ ranging from 2 mm to 40 mm.

Preferably, the distance between the first electrode 110 and the second electrode is substantially 10 mm.

According to the embodiment as shown in the example of Figure 2, the apparatus 1 comprises a reservoir 120 installed in the base body 105 for containing skin treatment substances. The reservoir 120 is in fluid communication with at least one hole or cavity 121 located at the terminal end 105a of the base body 105, for discharge of the curative substances.

In practice, once the electric field E to be applied into the portion of the patient's skin for cell membrane permeabilization, the desired curative substances may be applied into the skin, such that the substance may penetrate the skin without causing any trauma to the patient.

In one version, the hole/cavity 121 is placed between the first electrode 110 and the second electrode 111 of the applicator 100.

In one embodiment, the applicator 100 comprises a substance dispensing device 130 having the shape of a cylinder that projects out of the terminal end 105a of the base body 105. In a preferred embodiment, the cylinder may have one or more cavities 121 arranged over the surface, and in fluid connection with one another. Particularly, the cylinder may have an inlet cavity 131 in fluid communication with the reservoir 120, which branches out into one or more outlet cavities 132 for dispensing the curative substance.

Preferably, the cylinder is placed between the first electrode 110 and the second electrode 111 of the applicator 100.

In order to discharge the substances from the applicator 100, pumping means 122 may be provided, associated with the reservoir 120. In one version, the pumping means 122 may include a piston/cylinder system installed in the reservoir 120. Preferably, the reservoir 120 and the pumping means 122 are entirely held in the base body 105.

A skilled person will obviously appreciate that the pumping means may be different from those illustrated herein, in terms of shape, structure and size. Different pumping means may be also used for discharging the substances from the applicator, according to the applied electric field E.

For this purpose, the applicator 100 may be equipped with drive means 125 connected to the pumping means 122 for driving the pumping means and control means (not shown) for controlling the amount of substances to be discharged by the applicator 100.

Preferably, the drive means 125 are held within the base body 105.

In a further version, the drive means 125 are held within the reservoir 120.

Advantageously, the apparatus of the invention comprises insulating means (not shown) for electrically insulating the electrodes 110, 111 from the substance discharging hole 121. This will prevent circulation of currents between the pair of electrodes 110, 111 and the hole 121, that might cause changes in the electric field E, caused by the resistance of the curative substance itself.

In one version, the base body 105 is made of an insulating material.

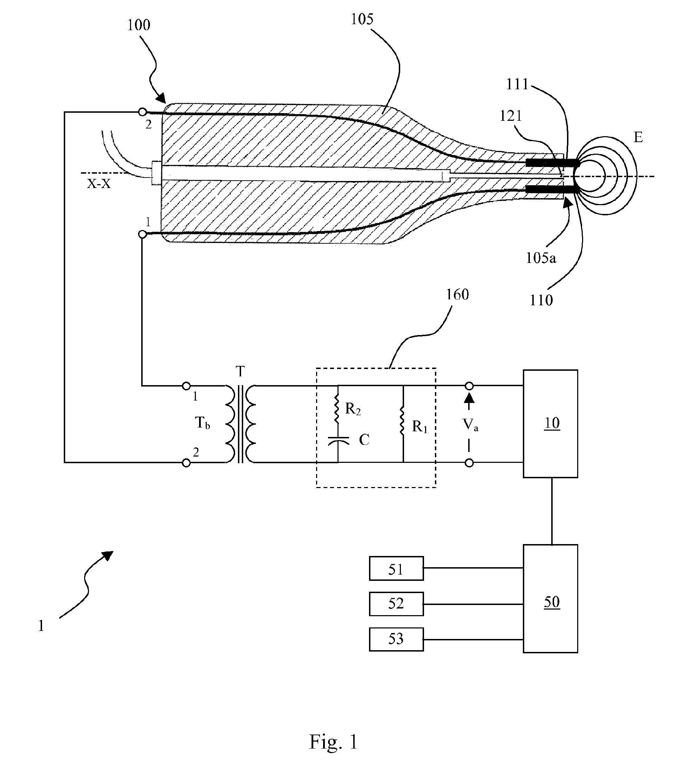

According to the embodiment as shown in the example of Figure 1, the power source 10 is adapted to generate a pulsed square-wave voltage Vₐ ranging from 0 to 200 Volt.

In one version, the power source is adapted to generate electric pulses having a duration ranging from 10 microseconds to 100 milliseconds. Preferably, the pulses have a duration of 1 millisecond.

The electric pulses may be generated at a frequency ranging from 0.5 Hz to 1000 Hz.

Still referring to the example as shown in Figure 1, the apparatus 1 comprises a load 160 for controlling the voltage and/or current that is designed to reach the applicator 100.

In one version, the load 160 has at least one resistor R₁,R₂ and at least one capacitor C, which are connected in parallel with the transformer T.

Preferably, a first resistor R₁ is connected in parallel with the output of the power source 10. A resistive/capacitive load is provided upstream from the resistor R₁, and consists of the series-connection of a second resistor R₂ and the capacitor C. According to the embodiment as shown in the example of Figure 1, the apparatus 1 is equipped with a plurality of sensors for measuring pH, temperature and humidity. Particularly, a first sensor 51 might be provided for measuring the pH value of the skin of the patient to be treated and a second 52 and a third 53 sensor might be provided for measuring ambient temperature (e.g. in °C) and humidity proximate to the patient.

The apparatus 1 also has processing means 50 for receiving the pH, temperature and humidity values from the sensors 51, 52, 53. Advantageously, the processing means 50 connected to the power source 10 are designed to change the pulsed voltage value Vₐ according to the measured pH, ambient temperature and humidity values.

In one version, the processing means 50 are in signal communication with the drive means 125 and are designed to control the drive means 125 according to the measured pH, temperature and humidity values. Thus, the amount of curative substance dispensed by the applicator is advantageously controlled according to the measured pH, temperature and humidity values.

According to one embodiment, the processing means 50 are also adapted to receive the value of the electrical resistance of skin TEER /Trans-Epithelial Electrical Resistance). For this purpose, the apparatus 1 of the invention has means for measuring the electrical resistance of the skin (TEER) of a user.

The processing means 50 are in signal communication with the power source 10 and are designed to receive and consequently change the value of pulsed voltage Vₐ according to the measured value of electrical resistance of the skin TEER.

In one version, the processing means 50 are in signal communication with the drive means 125 and are designed to receive and consequently change the value of pulsed voltage Vₐ according to the measured value of electrical resistance of the skin TEER. Thus, the amount of curative substance dispensed by the applicator is advantageously controlled according to the measured value of electrical resistance of the skin TTER. Hence, the value of the reduced electrical resistance of skin (TEER) of the user may be reduced to allow the passage of skin care substances having a molecular weight ranging from 200 to 1000.

It may be appreciated from the above that the apparatus of the present invention can fulfill the requirements and obviate the prior art drawbacks as mentioned in the introduction of the present invention.

The invention also allows transcutaneous penetration of curative substances through the skin without causing trauma to the patient, by simply modifying skin permeability and allowing the passage of curative substances possibly having a high molecular weight.

Those skilled in the art will obviously appreciate that a number of changes and variants may be made to the apparatus of the invention as described hereinbefore to meet incidental and specific needs, without departure from the scope of the invention, as defined in the following claims.

## Claims

1. An apparatus (1) for cosmetic skin treatment of a patient, comprising:
- an applicator (100) having at least one base body (105) mainly extending in a longitudinal direction (X-X) and equipped with a first (111) and a second (110) unpolarized electrodes, each adapted to contact an area of the patient's skin to be treated, to apply a pulsed electric field (E) into a predetermined skin volume,
- a power source (10) connected to said electrodes (110, 111) for generating pulsed voltage (Vₐ) to generate said electric field (E),
- a transformer (T) connected between said electrodes (110, 111) and said power source (10), said transformer (T) having a first terminal of the secondary winding (T_{b}) in direct signal communication with the first electrode (110) of said applicator (100) and a second terminal of the secondary winding (T_{b}) in direct signal communication with the second electrode (111) of said applicator (100),
**characterized in that** said base body (105) tapers toward the electrodes (110, 111) and comprises:
- a reservoir (120) for containing skin care substances,
- pumping means (122) associated with said reservoir (120) for discharging said substances from said applicator (100),
**and in that** said reservoir (120) and said pumping means (122) are entirely held in said base body (105).

2. An apparatus (1) as claimed in claim 1, wherein said first electrode (110) is located at a distance of 2 mm to 40 mm from said second electrode (111).

3. An apparatus (1) as claimed in claim 1 or 2, wherein said base body (105) comprises a substance dispensing device (130) located at said terminal end (105a) of said base body (105) between said first electrode (110) and said second electrode (111), wherein said substance dispensing device (130) has an inlet cavity (131) in fluid communication with said reservoir (120), which branches out into one or more outlet cavities (132) for dispensing the curative substance.

4. An apparatus (1) as claimed in any claim from 1 to 3, wherein said pumping means (122) comprise a piston/cylinder system associated with said reservoir (120) and entirely held win said base body (105) for discharging said substances from said applicator (100).

5. An apparatus (1) as claimed in any one of claims 1 to 4, wherein said power source (10) is adapted to generate electric pulses having a duration ranging from 10 microseconds to 100 milliseconds.

6. An apparatus (1) as claimed in any claim from 1 to 5, wherein said applicator (100) comprises drive means (125) connected to said pumping means (122) for driving said pumping means (122),
and wherein said drive means (125) are entirely held in said base body (105).

7. An apparatus (1) as claimed in claim 6, comprising:
- a first sensor (51) for measuring the pH value of the skin of said patient,
- a second (52) and a third (53) sensors, for measuring ambient temperature and humidity proximate to said patient respectively,
- processing means (50) for receiving said pH, temperature and humidity values from said first (51), second (52) and third (53) sensors respectively, said processing means (50) being in signal communication with said power source (10),
wherein said processing means (50) are designed to control said drive means (125) according to said measured pH, ambient temperature and humidity values.

8. An apparatus (1) as claimed in claim 6 or 7, comprising:
- means for measuring the electrical resistance of the skin (TTER) of a user,
- processing means (50) for receiving the value of said electrical resistance of skin (TEER), said processing means (50) being in signal communication with said power supply (10),
wherein said processing means (50) are designed to change control said drive means (125) according to said measured electrical resistance of skin (TEER), to reduce the value of said electrical resistance of skin (TEER) of the user.

9. An apparatus (1) as claimed in claim 8, wherein the value of said reduced electrical resistance of skin (TEER) allows the passage of skin care substances having a molecular weight ranging from 200 to 1000.
